Europäisches Patentamt

European Patent Office

Office européen des brevets

(19)

(11) Publication number: **0 301 423 B1**

(12) EUROPEAN PATENT SPECIFICATION

(45) Date of publication of patent specification: **27.04.94**  (51) Int. Cl.5: **A61K  9/22**, A61K 31/505

(21) Application number: **88111814.5**

(22) Date of filing: **22.07.88**

The file contains technical information submitted after the application was filed and not included in this specification

(54) **Pharmaceutical compositions containing trapidil, and process for their preparation.**

(30) Priority: **31.07.87 IT 2154087**

(43) Date of publication of application:
**01.02.89 Bulletin  89/05**

(45) Publication of the grant of the patent:
**27.04.94 Bulletin  94/17**

(84) Designated Contracting States:
**AT BE CH DE ES FR GB GR IT LI LU NL SE**

(56) References cited:
**EP-A- 0 142 877        EP-A- 0 156 592**
**EP-A- 0 187 433        EP-A- 0 205 336**
**EP-A- 0 207 638        GB-A- 2 163 648**
**US-A- 4 389 393**

(73) Proprietor: **CHIESI FARMACEUTICI S.p.A.**
**Via Palermo, 26/A**
**I-43100 Parma(IT)**

(72) Inventor: **Chiesi, Paolo**
**Via Palermo, 26/A**
**I-43100 Parma(IT)**
Inventor: **Pavesi, Luciana**
**Via Palermo, 26/A**
**I-43100 Parma(IT)**
Inventor: **Acerbi, Daniela**
**Via Palermo, 26/A**
**I-43100 Parma(IT)**

(74) Representative: **Minoja, Fabrizio**
**Studio Consulenza Brevettuale**
**Via Rossini, 8**
**I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 301 423 B1

**Description**

The present invention refers to a pharmaceutical composition for oral administration containing Trapidil and to a process for the preparation thereof.

Trapidil, i.e. 5-methyl-7-diethylamino-5-triazole-[1,5-a]-pyrimidine,is a substance having a wide spectrum of pharmacologic and biochemical activities on the circulatory system: dilatation of large coronary arteries and collateral pathways, development of inter-arteriolar coronary anastomosis, inhibition of phosphodiesterase, inhibition of platelet aggregation and inhibition of thromboxane $A_2$ synthesis.

It is indicated for the treatment of conditions of reduced artery perfusion and platelet hyper-aggregation, where a circulation improvement and an anti-thrombotic activity are required. In particular: ischaemic cardiopathy, chronic cerebral vasculopathies, consequences of myocardial infarct and of cerebral ictus.

After oral administration, Trapidil is quickly absorbed, reaching maximum plasmatic concentration 1 hour after administration. Hematic level then decreases rapidly, reducing to half in 90 minutes and after about 10 hours there is an almost total disappearance of the active principle from the blood.

The half-life period is equally short (1.5 - 1.7 hours).

The drug is quickly metabolized and eliminated through urines. Two metabolites derive from the loss of 1 or 2 ethyl residues (TP1 and TP2), of which the first reaches maximum serum concentration already 3 hours after administration, but it then decreases rapidly.

The TP1 metabolite is endowed with cardiovascular activity.

Studies conducted in mice show in fact that said compound causes an increase in the coronary flow and relaxation of the isolated coronary artery.

Fast metabolization of Trapidil and fast elimination of the TP1 metabolite cause a quick disappearance of the two active principles from the circle thus making frequent drug administrations necessary over the time.

This clearly causes upset to the patient, who often has to take other drugs at the same time, especially when considering the fact that it is a drug for long term treatments.

Therapeutic advantages deriving from the use of pharmaceutical preparations having a prolonged release of the active principle, regarding frequency of administration, reduction of side effects and maintenance of effective hematic concentration have been recognized.

Object of the present invention is an oral controlled release pharmaceutical composition containing Trapidil as active ingredient, which grants to the organism the right drug amount for an extended period of time sufficient to obtain the desired pharmacological response with a simplified posology (2 administrations/die).

A first object of the invention is an oral pharmaceutical composition containing as base carrier a mixture of hydroxypropylmethylcellulose and methylcellulose or other cellulose ethers characterized in that the solid carrier material is in an amount comprised between 30% and 50% by height of the active principle with the proviso that the amount of the cellulose ether is not less than 30% by weight of the mixture and with conventional excipients for tablets, such as lactose, magnesium stearate, silica or, in general, binding, lubricating, disintegrating agents and similar. Hydroxypropylmethylcelluloses are available in various grade under different trade names. The use of cellulose derivatives such as hydroxypropylmethylcellulose in pharmaceutical formulations has long been known, (US-B1-4389393).

EP-A-205336 and EP-A-207638 disclose sustained release pharmaceutical compositions comprising hydroxypropylmethylcellulose and a polymeric component which is reported to be critical for the release characteristics of the compositions.

GB-A-2163648 discloses "floating" compositions based on a combination of a solid fat or oil with a cellulose derivative.

EP-A-187433 discloses powder compositions suited for the nasal or buccal administrations of steroids or glycyrrizic acid derivatives, comprising cellulose ethers as carriers.

The properties of these cellulose ethers, obtained by substitution of cellulose hydroxyl with methoxy groups and by further introduction of hydroxypropyl groups, are a function of the substitution degree, distribution and type of the substituents, which determine solubility and viscosity degree. Therefore the selection of certain cellulose ethers or their mixtures as base carrier is quite important in the preparation of controlled release pharmaceutical compositions. Cellulose ethers such as hydroxypropylmethylcellulose are hydrophilic and tend to absorb water from the surrounding environment. For an effective controlled release of the active principle, hydration degree and rate of the matrix, expressed also as viscosity degree, must be evaluated in correlation to the solubility of the drug. Other elements influencing the release pattern of the active ingredient are the composition of the carrier base material, its weight ratio with the active principle and the degree of compression of the mixture. It has now been found, and it is the object of the present invention, that controlled release pharmaceutical compositions of Trapidil can be obtained when using as a carrier matrix a mixture of a hydroxyprophylmethylcellulose containing an average of 22.1% by weight of methoxy groups and an average of 8.1% by weight of hydroxypropoxy groups and whose viscosity degree (viscosity of 2% aqueous solution at 20°C) is comprised between $10^{-2}$ Ns/m$^2$ and 100 Ns/m$^2$ (10 and 100,000 centipoises), selected from the commercially known ones such as Methocel® K 100, K 4 M, K 15 M or K 100 M, combined with another cellulose ether such as methylcellulose, in an amount not less than 30% of the mixture. Particularly advantageous has proven to be furthermore the addition to the composition of an acidifying agent consisting of an organic acid such as tartaric, malic, maleic, malonic or citric acid (preferably citric acid) to provide, when the active principle is released, a microenvironment with acid pH, thus reproducing the most favourable conditions for absorption of the drug that is rapidly absorbed at gastric level from conventional compositions. The preparation of the pharmaceutical compositions of the invention is simply made by direct compression of the components, previous granulation of the mixture of the active principle plus citric acid, if present.

A second aspect of the invention refers to a process for the preparation of oral, controlled release pharmaceutical compositions containing Trapidil.

Spray-dried lactose, hydroxypropylmethylcellulose, optionally combined with methylcellulose, and magnesium stearate are added to a mixture of Trapidil and silica suitably sieved. The various ingredients are mixed and then compressed.

When citric acid is added, a mixture of this and Trapidil is prepared and is then granulated with water. The pre-sieved silica and the other components are added to the sieved and dried granulate. All the ingredients are mixed and then compressed.

The examples wich follow illustrate the invention in more details without limiting it in any way.

The amounts indicated in the examples are for the preparation of 10,000 tablets. The active principle dosage can vary between 100 and 300 mg per unit: the examples refer to a unit dose of 200 mg.

## EXAMPLE 1

Preparation of tablets containing 200 mg of active principle with no addition of citric acid.

2.0 Kg of Trapidil and 0.020 Kg of precipitated silica were placed into a stainless steel cubical mixer of a 8 l total capacity rotating at a speed of 25 r.p.m. The mixture was stirred for 10 minutes and it was then sieved thus obtaining a powder which was placed in a stainless steel cubical mixer of a capacity of 18 l and 0.85 Kg of spray-dried lactose, 0.45 Kg of Methocel® K100 and 0.45 Kg of Methocel® A4C (methylcellulose) were added. It was rotated at a speed of 25 r.p.m. for 10 minutes.

0.03 Kg of magnesium stearate previously sieved were added to the mixture and preparation of the tablets was carried out by compression of the mixture through rotating press.

| Dosage unit composition | |
| --- | --- |
| Trapidil | 200 mg |
| Methocel® K100 | 45 mg |
| Methocel® A4C | 45 mg |
| Spray-dried lactose | 85 mg |
| Silica | 2 mg |
| Magnesium stearate | 3 mg |
| Tablet total weight | 380 mg |

## EXAMPLE 2

Preparation of tablets containing 200 mg of active principle with addition of citric acid.

2 Kg of Trapidil and 0.65 Kg of citric acid suitably ground and sieved were placed in a granulator-mixer and mixed for 10 minutes.

Granulation of the mixture thus obtained was then carried out with H$_2$O. The granulate was dried in an air-circulation stove, sieved through a suitable sieve and then mixed with 0.45 Kg of Methocel® K100, 0.45 Kg of Methocel® A4C, 0.2 Kg of spray-dried lactose and 0.02 Kg of previously sieved silica, using a stainless steel cubical mixer of a 18 l capacity.

It was stirred for 10 minutes at 25 r.p.m. 0.03 Kg of magnesium stearate previously sieved was added to the mixture and preparation of the tablets was carried out by compression of the mixture through a rotating press.

| Dosage unit composition | |
|---|---|
| Trapidil | 200 mg |
| Methocel® K100 | 45 mg |
| Methocel® 4C | 45 mg |
| Spray-dried lactose | 20 mg |
| Silica | 2 mg |
| Magnesium stearate | 3 mg |
| Citric acid | 65 mg |
| Tablet total weight | 380 mg |

**In vitro release pattern of compositions prepared according to the Examples.**

Dissolution measurements were effected according to USP XXI method at 37°C and paddle speed of 50 r.p.m..The dissolution medium was simulated intestinal fluid at pH 6.8.

Results of cumulative percentage release referred to the contents:

| Time (hours) | Composition prepared according to example 1 | Composition prepared according to example 2 (containing citric acid) |
|---|---|---|
| 1 | 22.6 | 28.4 |
| 2 | 33.7 | 42.7 |
| 4 | 48.9 | 68.3 |
| 6 | 65.4 | 88.7 |
| 8 | 76.8 | 101.5 |

The above data show that the two formulations present a good in vitro release profile.

The addition of citric acid brings a sensible increase of the release of the active principle.

**In vivo bioavailability tests**

Bioavailability and pharmacokinetic profiles of the pharmaceutical compositions described in examples 1 and 2, administered in unit doses containing 200 mg of Trapidil, were evaluated in 4 adult healthy volunteers, aged 20 to 50, of normal blood pressure and good physical conditions, avoiding the intake of other drugs.

Each subject fasting, took each test composition, with an interval of 4 days between the two administration regimen and abstaining from food for 2 hours after each administration.

Blood samples to determine concentration of the active principle were collected as follows: 30 minutes; 1 hour; 2 hour; 4 hour; 6 hour; 8 hour; 12 hour.

The heparinized blood samples were centrifuged within 1 hour from sampling for 15 minutes at 1000xg and the separated plasma was kept under freezing at -18°C until the moment the active principle contents were assessed through HPLC technique by spectrophotometric detector.

Table 1 shows the mean plasma concentrations time values expressed in $\mu$g/ml and the main pharmacokinetic parameters derived from plasma concentrations of both Trapidil and its main circulating metabolite.

The results obtained with the compositions of examples 1 and 2 are compared with the results obtained in a different session, but under the same test conditions, with an equivalent dose of active principle administered in a composition at prompt release (2 tablets x 100 mg).

## TABLE 1

Plasmatic kinetics of Trapidil and of its main metabolite in man following single oral administration of equal doses (200 mg) of 100 mg Trapidil tablets at prompt release and of 200 mg Trapidil tablets at sustained release prepared according to Example 1 (composition with no citric acid) and Example 2 (composition with citric acid) of the present invention.

| FORM | | PLASMA CONC. (µg/ml) (X+S.E.) AT VARIOUS TIMES | | | | | | C.MAX (mcg/ml) | T.MAX (h) | AUC(0-12h) (mcg/ml)*h |
|---|---|---|---|---|---|---|---|---|---|---|
| | | 1 h | 2 h | 4 h | 6 h | 8 h | 12 h | | | |
| TRAPIDIL PROMPT RELEASE | TRAPIDIL | 6.67 | 5.06 | 2.86 | 1.15 | 0.48 | 0.14 | 6.79 | 1.2 | 24.00 |
| | | 0.49 | 0.17 | 0.2 | 0.14 | 0.19 | 0.07 | 0.38 | 0.2 | 0.84 |
| | TP1 | 0.49 | 1.04 | 1.24 | 1.04 | 1.01 | 0.5 | 1.34 | 4.3 | 10.57 |
| | | 0.13 | 0.12 | 0.11 | 0.06 | 0.1 | 0.03 | 0.08 | 0.8 | 0.76 |
| | TRAPIDIL +TP1 | 7.07 | 6.09 | 4.1 | 2.19 | 1.49 | 0.56 | 8.13 | 1.2 | 34.33 |
| | | 0.6 | 0.25 | 0.15 | 0.17 | 0.28 | 0.04 | 0.46 | 0.2 | 1.12 |
| TRAPIDIL - COMPOSITION OF EX. 1 | TRAPIDIL | 1.67 | 3.57 | 3.33 | 1.74 | 0.82 | 0.25 | 3.77 | 3 | 20.09 |
| | | 0.09 | 0.26 | 0.48 | 0.29 | 0.27 | 0.13 | 0.25 | 0.6 | 1.84 |
| | TP1 | 0.41 | 1.1 | 1.71 | 1.72 | 1.79 | 1.01 | 1.96 | 6.5 | 16.29 |
| | | 0.01 | 0.11 | 0.12 | 0.17 | 0.27 | 0.2 | 0.15 | 1 | 1.40 |
| | TRAPIDIL +TP1 | 2.08 | 4.67 | 5.03 | 3.45 | 2.61 | 1.26 | 5.4 | 3.5 | 36.39 |
| | | 0.09 | 0.32 | 0.58 | 0.43 | 0.5 | 0.3 | 0.33 | 0.5 | 3.26 |
| TRAPIDIL - COMPOSITION OF EX. 2 | TRAPIDIL | 2.95 | 3.66 | 3.46 | 1.18 | 0.75 | 0.26 | 3.78 | 2.3 | 20.51 |
| | | 0.24 | 0.23 | 0.33 | 0.1 | 0.02 | 0.01 | 0.19 | 0.6 | 1.15 |
| | TP1 | 0.65 | 1.21 | 2.12 | 2.51 | 2.36 | 1.81 | 2.62 | 6.5 | 22.41 |
| | | 0.06 | 0.09 | 0.12 | 0.22 | 0.04 | 0.03 | 0.15 | 0.5 | 0.85 |
| | TRAPIDIL +TP1 | 3.6 | 4.87 | 5.58 | 3.7 | 3.12 | 2.06 | 5.67 | 3.5 | 42.91 |
| | | 0.3 | 0.3 | 0.42 | 0.32 | 0.05 | 0.02 | 0.34 | 0.5 | 1.93 |

**TABLE 1 - continues**

```
S.E.    = Mean Standard Error
C.MAX   = Plasmatic concentration of Trapidil and of its
          main metabolite at the time T. MAX;
T.MAX   = Time when maximum plasmatic concentration of
          Trapidil and of its main metabolite was observed;
Trapidil prompt release n=6          (n = Number of subjects
Trapidil sustained release comp.n=4    partecipating    in
TP 1 = main metabolite                  the study)
Trapidil + TP 1 =      Values obtained by adding to plasma
                       concentrations of the active princi-
                       ple (Trapidil) those of the main
                       metabolite.
```

The analysis of the results of the Trapidil plasma kinetics show that, following administration of sustained release compositions, a remarkable reduction of the peak plasma concentration and an extended absorption were produced, so that the resulting plasma levels mantain higher than 3 $\mu$g/ml after the fourth hour from administration.

The advantages of the sustained release compositions of the invention are even more evident when comparing the cumulative data obtained by adding Trapidil plasma concentrations to the ones of its main metabolite.

In fact, the controlled release of the active principle causes an equally gradual release of its metabolite in the blood to keep till the 12th hour hematic levels of the active components higher than 1 $\mu$g/ml, which is the lowest concentration threshold to produce a therapeutic effect.

The pharmacokinetic profil of the composition of example 2, which contains citric acid in addition to other components, is even more favourable as:

1. after 1 hour from administration the Trapidil plasma values are higher than 3 $\mu$g/ml;

2. plasma levels of the active metabolite sensibly increase being superior or equal to 2 $\mu$g/ml until the 12th hour.

The evaluation of the bioavailability of sustained release compositions in comparison to standard release ones, carried out by comparing the respective areas under the plasma concentration/time curves (AUC), calculated using the trapezoidal rule, indicates a loss of bioavailability, as it does frequently happens in the sustained release compositions.

However, such bioavailability loss is limited to about 20% values.

In a subsequent study carried out under conditions as previously described, it was also established that food does not influence the absorption of Trapidil from the compositions object of the invention. The drug can thus be taken in said formulations with no need for particular care.

On the base of the results obtained following acute administration of standard and sustained release compositions of Trapidil, a simulation of plasma levels at the steady state was carried out, using a two compartments open model according to the procedure described in Notari R.E., Biopharmaceutics and Clinical Pharmacokinetics, III° ed., Marcel Dekker Inc.

The results, shown in table 2, further prove that, while the conventional composition allows to keep the minimum concentration of 1 $\mu$g/ml of Trapidil and of its active metabolite at the most until the 6th hour after administration, the compositions of the invention (and in particular the acidified composition described in example 2) secure effective minimal hematic levels of the active components until the 12th hour.

TABLE 2

| Cumulative simulated plasma levels of Trapidil and of its active metabolite (TP 1) in man at the steady state following oral administration of equivalent doses (300 mg/die) of prompt release composition and sustained release compositions of Trapidil prepared according to examples 1 and 2 of the invention. | | | | | | | |
|---|---|---|---|---|---|---|---|
| FORM | TRAPIDIL AND TP1 PLASMA CONC.($\mu$g/ml) AT VARIOUS TIMES (hours) | | | | | | |
| | basal | 1 | 2 | 4 | 6 | 8 | 12 |
| TRAPIDIL prompt release | 0.62 | 3.56 | 3.14 | 1.79 | 1.06 | 0.62 | |
| TRAPIDIL composition of example 1 | 1.00 | 3.07 | 4.20 | 3.71 | 2.86 | 2.10 | 1.00 |
| TRAPIDIL composition of example 2 | 1.66 | 4.19 | 4.90 | 4.85 | 3.88 | 2.96 | 1.66 |

The results confirm therefore the validity of the compositions of the invention, which ensure the presence in the organism of plasma concentrations of the drug and of its active metabolite sufficient to produce and keep the desired therapeutic effects by means of a simplified dosage scheme.

**Claims**

**Claims for the following Contracting States : AT, BE, CH, DE, FR, GB, IT, LI, LU, NL, SE**

1. Sustained release pharmaceutical compositions for oral administration, containing Trapidil as the active principle, optionally an organic acid selected from tartaric, malic, maleic, malonic and citric acid and conventional excipients, in combination with a solid carrier material consisting of a mixture of hydroxypropylmethylcellulose, containing an average of 22.1% by weight of methoxy groups and an average of 8.1% by weight of hydroxypropoxy groups and whose viscosity degree is comprised between $10^{-2}$ and 100 N s/m$^2$ (10 and 100,000 centipoises) with another cellulose ether, characterized in that the solid carrier material is in an amount comprised between 30% and 50% by weight of the active principle, with the proviso that the amount of said another cellulose ether is not less than 30% by weight of the mixture.

2. Pharmaceutical compositions according to claim 1 wherein the solid carrier material consists of a mixture of hydroxypropylmethylcellulose and methylcellulose and citric acid.

3. Pharmaceutical compositions according to claims 1-2, whose unit dosage form is a tablet containing an amount of active principle comprised between 100 and 300 mg.

4. A process for the preparation of pharmaceutical compositions according to claims 1-3 consisting in the direct compression of a mixture of the active principle with 30-50% by weight with respect to the active principle of a solid carrier material consisting of a mixture of hydroxypropylmethylcellulose, an average of 22.1% by weight of methoxy groups and an average of 8.1% Or weight of hydroxypropoxy groups and whose viscosity degree is comprised between $10^{-2}$ and 100 N s/m (10 and 100,000 centipoises) with another cellulose ether, wherein the amount of said another cellulose ether is not less than 30% by weight of said mixture, and with other conventional excipients, previous possible granulation of a mixture of the active principle with an organic acid selected from tartaric, malic, maleic, malonic and citric acid.

5. A process according to claim 4 characterized in that a granulate of a mixture of the active principle with citric acid is prepared and that said granulate is subsequently mixed and compressed with the solid carrier material previously described.

**Claims for the following Contracting States : ES, GR**

1. A process for the preparation of sustained release pharmaceutical compositions for oral administration, containing Trapidil as the active principle in combination with a solid carrier material consisting of a mixture of hydroxypropylmethylcellulose containing an average of 22.1% by weight of methoxy groups and an average of 8.1% by weight of hydroxypropoxy groups and whose viscosity rate is comprised between $10^{-2}$ and 100 Ns/m$^2$ (10 and 100,000 centipoises) with another cellulose ether in an amount not less than 30% by weight of said mixture, optionally in combination with an organic acid selected from tartaric, malic, maleic, malonic and citric acid, preferably citric acid, and with conventional excipients, which process consists in the direct compression of a mixture of the active principle with a solid carrier material consisting of a mixture of said hydroxypropylmethylcellulose with said another cellulose ether in an amount comprised between 30% and 50% by weight of the active principle, and with other conventional excipients, previous possible granulation of a mixture of the active principle with said organic acid.

2. A process according to claim 1 characterized in that a granulate of a mixture of the active principle with citric acid is prepared and that said granulate is subsequently mixed and compressed with the solid carrier material previously described.

**Patentansprüche**
**Patentansprüche für folgende Vertragsstaaten : AT, BE, CH, DE, FR, GB, IT, LI, LU, NL, SE**

1. Arzneimittel mit verzögerter Freisetzung für die orale Verabreichung, enthaltend Trapidil als den wirksamen Bestandteil, gegebenenfalls eine organische Säure, ausgewählt aus Weinsäure, Hydroxybernsteinsäure, Maleinsäure, Malonsäure und Zitronensäure und üblichen Trägern, in Kombination mit einem festen Trägermaterial, bestehend aus einem Gemisch von Hydroxypropylmethylcellulose, die durchschnittlich 22,1 Gew.-% Methoxygruppen und durchschnittlich 8,1 Gew.-% Hydroxypropoxygruppen enthält und deren Viskositätsgrad zwischen $10^{-2}$ und 100 N s/m$^2$ (10 und 100.000 Centipoise) mit einem anderen Celluloseether beträgt, dadurch **gekennzeichnet,** daß das feste Trägermaterial in einer Menge zwischen 30 und 50 Gew.-% des aktiven Bestandteils vorliegt, mit der Maßgabe, daß die Menge des anderen Celluloseethers nicht weniger als 30 Gew.-% des Gemisches beträgt.

2. Arzneimittel nach Anspruch 1, dadurch **gekennzeichnet,** daß das feste Trägermaterial aus einem Gemisch von Hydroxypropylmethylcellulose und Methylcellulose und Zitronensäure besteht.

3. Arzneimittel nach den Ansprüchen 1-2, dadurch **gekennzeichnet,** daß dessen Einheitsdosierungsform eine Tablette ist, die eine Menge des wirksamen Bestandteils zwischen 100 und 300 mg enthält.

4. Verfahren zur Herstellung eines Arzneimittels nach den Ansprüchen 1-3, dadurch **gekennzeichnet,** daß es aus der direkten Verpressung von einem Gemisch des aktiven Bestandteils mit 30-50 Gew.-% bezüglich des wirksamen Bestandteils eines festen Trägermaterials, bestehend aus einem Gemisch von Hydroxypropylmethylcellulose mit durchschnittlich 22,1 Gew.-% Methoxygruppen und durchschnittlich 8,1 Gew.-% Hydroxypropoxygruppen und deren Viskositätsgrad zwischen $10^{-2}$ und 100 N s/m$^2$ (10 und 100.000 Centipoises) beträgt, mit einem anderen Celluloseether, worin die Menge des anderen Celluloseethers nicht weniger als 30 Gew.-% des Gemisches beträgt, und mit anderen üblichen Trägern besteht, wobei zuvor eine Granulierung aus einem Gemisch des wirksamen Bestandteils mit einer organischen Säure, ausgewählt aus Weinsäure, Hydroxybernsteinsäure, Maleinsäure, Malonsäure und Zitronensäure, möglich ist.

5. Verfahren nach Anspruch 4, dadurch **gekennzeichnet,** daß ein Granulat aus einem Gemisch des wirksamen Bestandteils mit Zitronensäure hergestellt wird und daß das Granulat anschließend mit dem vorstehend beschriebenen festen Trägermaterial vermischt und verpreßt wird.

**Patentansprüche für folgende Vertragsstaaten : ES, GR**

1. Verfahren zur Herstellung eines Arzneimittels mit verzögerter Freisetzung für die orale Verabreichung, enthaltend Trapidil als den wirksamen Bestandteil in Verbindung mit einem festen Trägermaterial, bestehend aus einem Gemisch von Hydroxypropylmethylcellulose, die durchschnittlich 22,1 Gew.-%

Methoxygruppen und durchschnittlich 8,1 Gew.-% Hydroxypropoxygruppen enthält und deren Viskositätsgrad zwischen $10^{-2}$ und 100 N s/m$^2$ (10 und 100.000 Centipoises) beträgt, mit einem anderen Celluloseether in einer Menge von nicht weniger als 30 Gew.-% des Gemisches, gegebenenfalls in Kombination mit einer organischen Säure, ausgewählt aus Weinsäure, Hydroxybernsteinsäure, Maleinsäure, Malonsäure und Zitronensäure, vorzugsweise Zitronensäure, und mit den üblichen Trägern, dadurch **gekennzeichnet,** daß das Verfahren aus einer direkten Verpressung von einem Gemisch des wirksamen Bestandteils mit einem festen Trägermaterial, bestehend aus einem Gemisch der Hydroxypropylmethylcellulose, mit dem anderen Celluloseether in einer Menge zwischen 30 und 50 Gew.-% des wirksamen Bestandteils, und mit anderen üblichen Trägern besteht, wobei zuvor die Granulierung aus einem Gemisch des wirksamen Bestandteils mit der organischen Säure möglich ist.

2. Verfahren nach Anspruch 1, dadurch **gekennzeichnet,** daß ein Granulat aus einem Gemisch des wirksamen Bestandteils mit Zitronensäure hergestellt wird und daß das Granulat anschließend mit dem vorstehend beschriebenen festen Trägermaterial gemischt und verpreßt wird.

## Revendications
## Revendications pour les Etats contractants suivants : AT, BE, CH, DE, FR, GB, IT, LI, LU, NL, SE

1. Compositions pharmaceutiques à libération prolongée pour administration orale, contenant du Trapidil en tant que principe actif, facultativement un acide organique choisi parmi les acides tartrique, malique, maléique, malonique et citrique, et des excipients conventionnels, en association avec une matière de support solide consistant en un mélange d'hydroxypropylméthylcellulose, contenant en moyenne 22,1% en poids de groupes méthoxy et en moyenne 8,1% en poids de groupes hydroxypropoxy et dont le degré de viscosité est compris entre $10^{-2}$ et 100 N s/m$^2$ (10 et 100.000 centipoises) et d'un autre éther de cellulose, caractérisé en ce que la matière de support solide est présente en une quantité comprise entre 30% et 50% en poids du principe actif, avec la condition que la quantité dudit autre éther de cellulose n'est pas inférieure à 30% en poids du mélange.

2. Compositions pharmaceutiques selon la revendication 1 dans lesquelles la matière de support solide consiste en un mélange d'hydroxypropylméthylcellulose, de méthylcellulose et d'acide citrique.

3. Compositions pharmaceutiques selon les revendications 1-2, dont la présentation unitaire est un comprimé contenant une quantité de principe actif comprise entre 100 et 300 mg.

4. Procédé de préparation de compositions pharmaceutiques selon les revendications 1-3 consistant en la compression directe d'un mélange du principe actif et de 30-50% par rapport au principe actif d'une matière de support solide consistant en un mélange d'hydroxypropylméthylcellulose, contenant en moyenne 22,1% en poids de groupes méthoxy et en moyenne 8,1% en poids de groupes hydroxypropoxy et dont le degré de viscosité est compris entre $10^{-2}$ et 100 N s/m (10 et 100.000 centipoises) et d'un autre éther de cellulose, dans lequel la quantité dudit autre éther de cellulose n'est pas inférieure à 30% en poids dudit mélange, et avec d'autres excipients conventionnels, la granulation préalable éventuelle d'un mélange de principe actif et d'un acide organique choisi parmi les acides tartrique, malique, maléique, malonique et citrique.

5. Procédé selon la revendication 4 caractérisé en ce qu'un granulé d'un mélange de principe actif et d'acide citrique est préparé et subséquemment mélangé et comprimé avec la matière de support solide décrite précédemment.

## Revendications pour les Etats contractants suivants : ES, GR

1. Un procédé de préparation de compositions pharmaceutiques à libération prolongée pour administration orale, contenant du Trapidil en tant que principe actif, en association avec une matière de support solide consistant en un mélange d'hydroxypropylméthylcellulose, contenant en moyenne 22,1% en Poids de groupes méthoxy et en moyenne 8,1% en Poids de groupes hydroxypropoxy et dont le degré de viscosité est compris entre $10^{-2}$ et 100 N s/m$^2$ (10 et 100.000 centipoises) et d'un autre éther de cellulose, en une quantité non inférieure à 30% en poids du mélange facultativement en association avec un acide organique choisi parmi les acides tartrique, malique, maléique, malonique et citrique, de préférence l'acide citrique et des excipients conventionnels, ledit procédé consistant à compresser directement un mélange du principe actif avec une matière de support solide consistant en un mélange de ladite kydroxypropylméthylcellusose avec ledit autre éther de cellulose en une quantité comprise entre 30% et 40% en poids du principe actif et avec d'autres excipients conventionnels, la granulation préalable éventuelle d'un mélange du principe actif et dudit acide organique.

2. Procédé selon la revendication 1 caractérisé en ce qu'un granulé d'un mélange de principe actif et d'acide citrique est préparé et en ce que ledit granulé est subséquemment mélangé et comprimé avec la matière de support solide décrite précédemment.